(19) Europäisches Patentamt
European Patent Office
Office européen des brevets

(11) Veröffentlichungsnummer: **0 315 097**
**A2**

(12) **EUROPÄISCHE PATENTANMELDUNG**

(21) Anmeldenummer: **88118073.1**

(22) Anmeldetag: **29.10.88**

(51) Int. Cl.⁴: **C07D 311/92 , C07F 7/18 , A61K 31/695**

Patentansprüche für folgenden Vertragsstaat: ES + GR.

(30) Priorität: **04.11.87 DE 3737353**

(43) Veröffentlichungstag der Anmeldung: **10.05.89 Patentblatt 89/19**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Khandelwal, Yatendra, Dr.**
**M-8, Hoechst Executive Quarters Darga Road**
**Mulund (West) Bombay 400 082(IN)**
Erfinder: **Kannan, Rajeshwari**
**101, Abhinnandan Sarvodaya Parshwanath Nagar**
**Nahur Mulund (West) Bombay 400 080(IN)**
Erfinder: **Lal, Bansi, Dr.**
**30 Advani Apartments**
**Mulund (West) Bombay 400 080(IN)**
Erfinder: **Dohadwalla, Alihussein Nomanbhai, Dr.**
**Noman Mansion, 139 C Cumball Hill**
**Bombay 400 036(IN)**
Erfinder: **Rajgopalan, Ramanujam, Dr.**
**203, B Wing Arthi Sarojini Naidu Road**
**Mulund (West) Bombay 400 080(IN)**
Erfinder: **Rupp, Richard Helmut, Dr.**
**Roederweg 16a**
**D-6240 Königstein/Taunus(DE)**

(54) **Neue Acyllabdan-Derivate, ein Verfahren zu ihrer Herstellung und ihre Verwendung als Arzneimittel.**

(57) Die vorliegende Erfindung betrifft Acyllabdan-Derivate der Formel

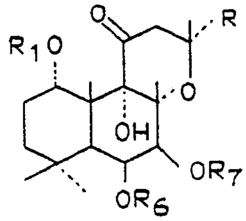

I

ein Verfahren zu ihrer Herstellung und die Anwendung der Substanzen als pharmazeutische Mittel, insbesondere als Medikamente mit Wirkung gegen erhöhten Augeninnendruck und erhöhten Blutdruck.

EP 0 315 097 A2

# Neue Acyllabdan-Derivate, ein Verfahren zu ihrer Herstellung und ihre Verwendung als Arzneimittel

Die vorliegende Erfindung betrifft Labdane der Formel I

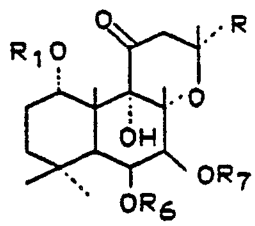

I

worin bedeuten

R Vinyl, Äthyl, Cyclopropyl oder $CHOHCH_2OH$, $R_1$ Wasserstoff, eine Gruppe der Formel $-\overset{\overset{O}{\|}}{C}-A$, wobei A $OR_2$, worin $R_2$ eine Alkylgruppe darstellt, oder

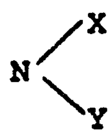

bedeutet,

wobei X und Y, falls sie gleich sind, Wasserstoff oder Alkyl darstellen, oder, falls X für Wasserstoff oder Niederalkyl steht, Y eine Alkyl-, substituierte Alkyl-, Cycloalkyl-, Aralkyl-, Aryl-, Amino- oder Hydroxylgruppe darstellt, oder X und Y mit dem Stickstoffatom, an das sie gebunden sind, einen heterocyclischen Ring bilden, der ein weiteres Heteroatom enthalten und durch eine Alkyl- oder Arylgruppe substituiert sein kann, oder für eine Gruppe der Formel $R_3R_4R_5Si$, wobei $R_3$, $R_4$ and $R_5$ unabhängig je eine Alkylgruppe bedeuten,

$R_6$ eine Gruppe der Formel

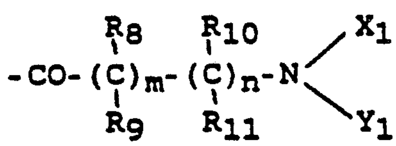

worin m und n ganze Zahlen von 0 bis 10 sind und $R_8$ und $R_9$ gleich oder verschieden sind und Wasserstoff oder eine Niederalkylgruppe darstellen, oder einer der Substituenten Wasserstoff und der andere eine Hydroxy-, Thio-, oder Arylgruppe darstellt, $R_{10}$ Wasserstoff und $R_{11}$ Wasserstoff oder eine Hydroxy- oder Alkylgruppe bedeuten, und $X_1$ Wasserstoff darstellt, falls $Y_1$ für Wasserstoff, Alkyl, substituiertes Alkyl, Alkanoyl, Aryl, Cycloalkyl, Aralkyl, einen Heterocyclus, Amino, substituiertes Amino, Hydroxy, Acyl, Dialkylaminoalkyl, Carbamoyl, Carboxyalkyl oder Carbalkoxyalkyl steht, oder $X_1$ und $Y_1$, falls sie gleich sind, Alkyl substituiertes Alkyl, Aryl oder Aralkyl darstellen oder, falls $X_1$ für Alkyl steht, $Y_1$ substituiertes Alkyl, Cycloalkyl, Aralkyl oder eine Dialkylaminoalkylgruppe darstellt, oder $X_1$ und $Y_1$ zusammen mit dem Stickstoffatom, an das sie gebunden sind, einen Heterocyclus bilden, der ein oder mehrere Heteroatome enthalten und gegebenenfalls ein- oder mehrfach durch Alkyl-, Aryl-, Aralkyl-, Hydroxyalkyl-, Hydroxyl- oder andere heterocyclische Gruppen substituiert sein kann,

$R_7$ eine Gruppe der Formel

$-\overset{\overset{O}{\|}}{C}-A$, worin A dieselbe Bedeutung wie oben für $R_1$ oder

$-\overset{\overset{O}{\|}}{C}-A$ angegeben hat, oder A eine Alkylgruppe bedeutet, wenn $R_6$ für eine Gruppe der Formel

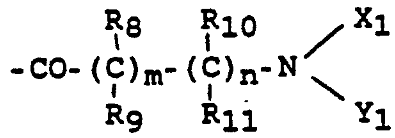

steht, worin $R_8$ bis $R_{11}$, $X_1$, $Y_1$, m und n die obigen Bedeutungen haben, sowie deren optische und geometrische Isomere und deren pharmazeutisch unbedenkliche Säureadditionssalze.

Bevorzugt sind Verbindungen der Formel I, worin R die oben angegebene Bedeutung hat und entweder

a) $R_1$ für Wasserstoff, $R_6$ für eine Gruppe der Formel

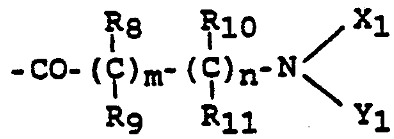

worin $R_8$, $R_{10}$, $R_{11}$, $X_1$ und $Y_1$ die oben angegebene Bedeutung haben, und $R_7$ für eine Gruppe der Formel $-\overset{O}{\overset{\|}{C}}-A$ stehen, wobei A einen Rest der Formel

$OR_2$ oder 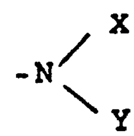

bedeutet, worin $R_2$, X und Y die oben angegebenen Bedeutungen haben, oder

b) $R_1$ und $R_7$ für eine Gruppe der Formel $-\overset{O}{\overset{\|}{C}}-A$ stehen, wobei A einen Rest der Formel

$-OR_2$ oder 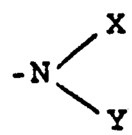

bedeutet, worin $R_2$, X und Y die oben angegebenen Bedeutungen haben und $R_6$ für eine Gruppe der Formel

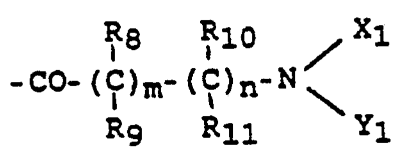

steht, worin $R_8$, $R_9$, $R_{10}$, $R_{11}$, $X_1$ und $Y_1$ die oben angegebenen Bedeutungen haben.

Der Begriff Alkyl bezieht sich auf geradkettige oder verzweigte gesättigte Kohlenwasserstoffreste mit 1 bis 8 Kohlenstoffatomen, wie z.B. Methyl, Ethyl, Propyl, 2-Methylpropyl, 1-Pentyl, 3-Hexyl oder 2-Octyl und dergleichen. Bevorzugt sind Alkylgruppen mit 1 bis 6, insbesondere mit 1 bis 4 C-Atomen.

Geeignete Beispiele für substituierte Alkylgruppen sind Hydroxyalkyl wie Hydroxyethyl, Carboxyalkyl wie Carboxyethyl und Carbalkoxyalkyl wie Carbethoxyethyl, oder halogeniertes Alkyl.

Geeignete Cycloalkylgruppen sind $C_3$-$C_7$-Cycloalkylgruppen, insbesondere Cyclopentyl oder Cyclohexyl.

Unter einer Aralkylgruppe ist zu verstehen eine Phenylalkylgruppe, bevorzugt Phenyl-$C_1$-$C_3$-alkyl, z.B. eine Benzylgruppe, worin die Phenylgruppe durch Halogen, $C_1$-$C_3$-Alkyl, $C_1$-$C_3$-Alkoxy, Nitro oder Trifluormethyl einfach oder mehrfach substituiert sein kann.

Unter einer Arylgruppe ist zu verstehen eine Phenylgruppe, welche durch Substituenten wie Halogen, $C_1$-$C_3$-Alkyl, $C_1$-$C_3$-Alkoxy, Nitro oder Trifluormethyl einfach oder mehrfach substituiert sein kann.

Unter einer Acylgruppe ist zu verstehen $C_1$-$C_6$-Alkanoyl, $C_2$-$C_6$-Alkenoyl, $C_3$-$C_6$-Alkinoyl, Aroyl, Aralkanoyl oder eine Heteroaroylgruppe mit bis zu 10 Kohlenstoffatomen, wobei ein oder mehrere C-Atome durch Sauerstoff, Stickstoff und/oder Schwefel ersetzt sein können.

Beispiele für Alkanoylgruppen sind Formyl, Acetyl, Propionyl, Butyryl, Isobutyryl, Valeryl, Palmityl und Bromisobutyryl. Die Alkanoylgruppen können eine oder mehrere Doppelbindungen enthalten, z.B. eine Acryloyl-, Stearoyl- oder Oleoylgruppe. Die Alkanoylgruppen können auch eine oder mehrere Dreifachbindungen und außerdem eine oder mehrere Doppelbindungen enthalten. Ein Beispiel für eine solche Alkinoylgruppe ist die Propiolylgruppe. Ein Vertreter für Aroylgruppen ist die Benzoylgruppe, worin die Phenylgruppe durch Substituenten wie $C_1$-$C_3$-Alkyl, $C_1$-$C_3$-Alkoxy, Halogen, Nitro und Trifluormethyl einfach oder mehrfach substituiert sein kann. Beispiele für Aralkanoyl-bzw. Heteroaroylgruppen sind Phenylacetyl- bzw. Pyridin-3-carbonylgruppen.

Unter Dialkylaminoalkylgruppen sind solche zu verstehen, in denen die Alkylgruppen jeweils 1 bis 6 C-Atome enthalten, z.B. Diethylaminoethyl.

Falls X und Y bzw. $X_1$ und $Y_1$ zusammen mit dem Stickstoffatom, an das sie gebunden sind, einen heterocyclischen Ring bilden, sind Piperidin, Pyrrolidin, Morpholin, Piperazin, Thiomorpholin, Imidazol und Theophyllin bevorzugt, die gegebenenfalls in einer oder mehreren Stellungen durch $C_1$-$C_4$-Alkyl, $C_1$-$C_4$-Alkoxy, Aryl, Aryl-$C_1$-$C_4$-alkyl, Hydroxy, Amino oder substituiertes $C_1$-$C_4$-Alkyl substituiert sein können.

Geeignete Beispiele für die Salze der erfindungsgemäßen Verbindungen mit anorganischen oder organischen Säuren sind das Hydrochlorid, Hydrobromid, Sulfat, Phosphat, Acetat, Oxalat, Tartrat, Citrat, Maleinat oder Fumarat.

In den hier abgebildeten Formeln sind die verschiedenen Substituenten als in einer von zwei Bezeichnungsweisen mit dem Labdankern verbunden dargestellt: Eine ausgezogene Linie (__), die einen Substituenten in der $\beta$-Orientierung (d.h. über der Ebene des Moleküls) und eine gestrichelte Linie (---), die einen Substituenten in der $\alpha$-Orientierung (d.h. unter der Ebene des Moleküls) angibt. Sämtliche Formeln sind so gezeichnet, daß sie die Verbindungen in ihrer absoluten stereochemischen Konfiguration veranschaulichen. Sofern die einen Labdankern aufweisenden Ausgangsstoffe natürlich vorkommen oder von Naturstoffen abgeleitet sind, besitzen sie wie die Endprodukte einen Labdankern in der hier abgebildeten, einzigen absoluten Konfiguration. Das erfindungsgemäße Verfahren ist jedoch ebenfalls zur Synthese von Labdanen der racemischen Serie bestimmt.

Zusätzlich zu den optischen Zentren des Labdankerns können die Substituenten darauf auch chirale Zentren aufweisen, die zu den optischen Eigenschaften der erfindungsgemäßen Verbindungen beitragen und deren Trennung nach herkömmlichen Methoden ermöglichen, beispielsweise durch die Anwendung optisch aktiver Säuren. Eine Wellenlinie (~), die eine Gruppe mit einem chiralen Zentrum verbindet, zeigt an, daß die Stereochemie des Zentrums unbekannt ist, d.h. die Gruppe kann in jeder der möglichen Orientierungen vorliegen. Diese Erfindung umfaßt sämtliche optischen Isomeren und racemischen Formen der erfindungsgemäßen Verbindungen, wo solche Verbindungen chirale Zentren zusätzlich zu denen des Labdankerns aufweisen.

Einige der erfindungsgemäßen neuen, mehrfach oxydierten Labdanderivate sind in der folgenden Tabelle 1 angeführt.

**Tabelle 1**

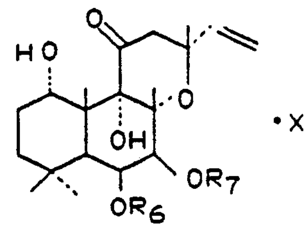

| $R_6$ | $R_7$ | X | Schmelzpunkt |
|---|---|---|---|
| $COCH_2N$ 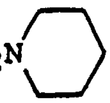 | $COOC_2H_5$ | HCl | 268°C |
| $COCH_2$-N 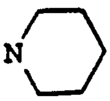 | $CON(C_2H_5)_2$ | HCl | 253°C |
| $COCH_2$-N 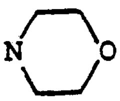 | $COOC_2H_5$ | $HCl \cdot H_2O$ | 225-29°C |
| $CO(CH_2)_2N(CH_3)_2$ | $COOC_2H_5$ | HCl | 213-15°C |
| $CO(CH_2)_2N(CH_3)_2$ | $CON(C_2H_5)_2)$ | HCl | 239-40°C |
| $CO(CH_2)_2N$ 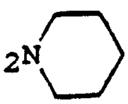 | $COOC_2H_5$ | HCl | 227-28°C |

| $R_6$ | $R_7$ | X | Schmelzpunkt |
|---|---|---|---|
| 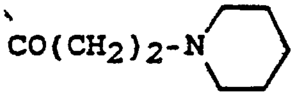 | $CON(C_2H_5)_2$ | HCl | 246-47°C |
| 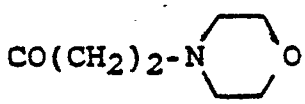 | $COOC_2H_5$ | HCl | 218-20°C |
| 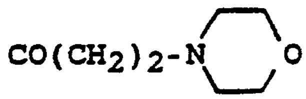 | $CON(C_2H_5)_2$ | $HCl \cdot 0{,}5\ H_2O$ | 160-64°C |
| 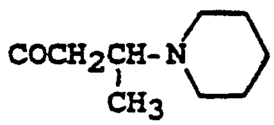 | $COOC_2H_5$ | HCl | 175-78°C |
| 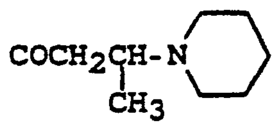 | $CON(C_2H_5)_2$ | $HCl \cdot H_2O$ | 173-75°C |
| 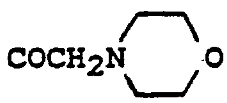 | $CONHC_6H_5$ | $HCl \cdot 1{,}5\ H_2O$ | 201-5°C |

Gegenstand der Erfindung ist ebenfalls ein Verfahren zur Herstellung der neuen Acyllabdane der Formel I, welches dadurch gekennzeichnet ist, daß man Verbindungen der Formel II

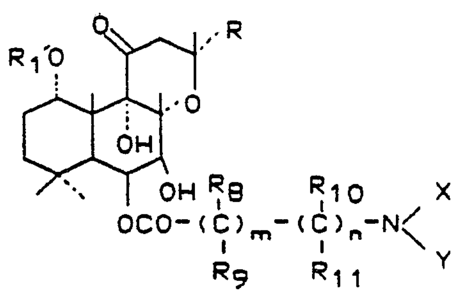

II

worin $R_1'$ eine Schutzgruppe für eine Hydroxylgruppe, wie z.B. t-Butyldimethylsilyl bedeutet und R, $R_8$-$R_{11}$ sowie $X_1$ und $Y_1$ die oben angegebenen Bedeutungen haben, mit einem Gemisch eines Halogenalkylformiates, z.B. Chlorethylformiat, und 4-Dimethylaminopyridin in organischen Lösungsmitteln wie Dichlormethan oder Essigester bei Temperaturen im Bereich von 20°C bis 70°C zu Verbindungen der Formel III,

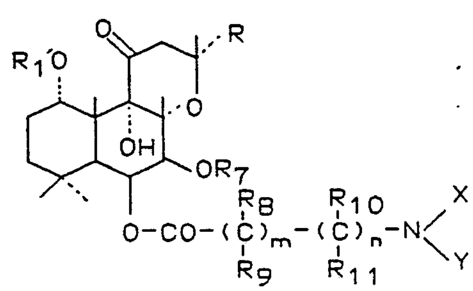

III

O

worin $R_1'$ für eine Schutzgruppe, $R_7$ für -C-$OR_2$ mit $R_2$ $C_1$-$C_8$-Alkyl stehen sowie R, $R_8$-$R_{11}$, $X_1$, $Y_1$, m und n die oben angegebenen Bedeutungen haben, umsetzt. Als Schutzgruppen für $R_1'$ kommen infrage: Methylether, t-Butylether, Allylether, Benzylether, Triarylmethylether, Trialkylsilylether, Tetrahydropyranylether oder Ester. Besonders vorteihaft wird als Schutzgruppe die t-Butyldimethylsilylgruppe verwendet. Um Verbindungen der Formel III, worin $R_7$ für

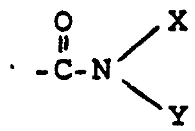

steht, zu erhalten, behandelt man die Verbindungen der Formel II mit dem entsprechenden Carbamoylchlorid der Formel

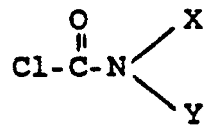

in Gegenwart von 4-Dimethylaminopyridin und Hydrochinonmonomethylether in einem organischen Lösungsmittel wie Pyridin bei einer Temperatur im Bereich von 20° C bis 30° C. Das Reaktionsprodukt der Formel III wird dadurch aus dem Gemisch gewonnen, daß man mit einem organischen Lösungsmittel extrahiert, die organische Schicht mit Wasser wäscht, über wasserfreiem Natriumsulfat trocknet und im Vakuum einengt. Die Reinigung erfolgt durch eine chromatographische Methode.

Verbindungen der Formel III, worin $R_1'$ für eine Schutzgruppe wie z.B. t-Butyldimethylsilyl steht, werden bei Temperaturen im Bereich von 0 bis 30° C mit Reagentien wie Tetrabutylammoniumfluorid behandelt, um zu entsprechenden Verbindungen der Formel I mit $R_1$ = H zu gelangen.

Verbindungen der Formel II werden nach dem Verfahren, das in EP-A-0 217 372 (HOE 85/F 214) beschrieben ist, aus Verbindungen der Formel IV

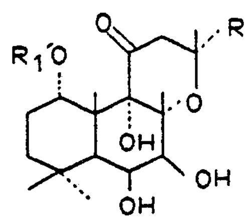

IV

worin $R_1$ für eine Schutzgruppe wie t-Butyldimethylsilyl und R für eine Vinylgruppe stehen, hergestellt.

Verbindungen der Formel IV werden nach der unten angegebenen Reaktionsfolge aus Forskolin (V) hergestellt.

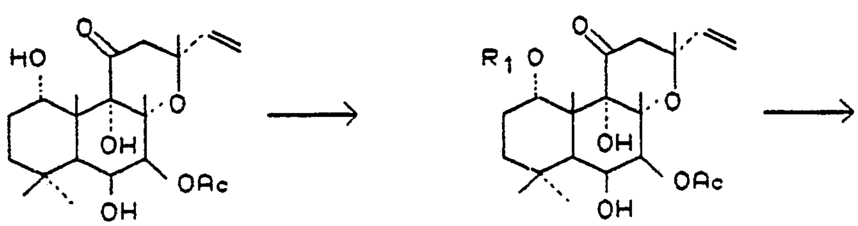

V VI

Die 1-OH-Gruppe in Forskolin (V) wird nach dem Fachmann bekannten Methoden (vgl. Reagents for Org. Synth., L.F. Fieser und M. Fieser, John Wiley & Sons, Band 1 bis 11) mit einer wie oben definierten Gruppe $R_1'$ geschützt.

Die Acetylgruppe in der 7-Stellung in Verbindungen der Formel VI wird durch alkalische Hydrolyse nach literaturbeschriebenen Methoden (vgl. J.C.S. Perkin I, 769 (1982)) abgespalten, was Verbindungen der Formel IV liefert.

Verbindungen der Formel I kann man ebenfalls ausgehend von Verbindungen der Formel IV erhalten.

Verbindungen der Formel IV, worin $R_1$ die obige Bedeutung hat, werden mit Carbonsäuren der Formel $R_{12}R_{13}C=CH\text{-}COOH$, worin $R_{12}$ und $R_{13}$ für Wasserstoff oder eine Alkyl- oder Arylgruppe stehen, in Gegenwart von 4-N-Dimethylaminopyridin und DCC in organischen Lösungsmitteln wie trockenem Dimethylformamid, trockenem Essigester usw. vier Stunden lang bei Temperaturen im Bereich von 20 bis 30 °C behandelt und das Produkt der Formel VII

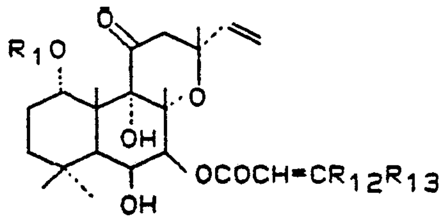

VII

worin $R_1$ und $R_{12}$ die obigen Bedeutungen haben, aus dem Reaktionsgemisch durch Verdünnen mit Wasser, nachfolgende Extraktion mit organischen Lösungsmitteln wie Essigester, Waschen des Extrakts mit Wasser, Trocknen über wasserfreiem Natriumsulfat und Einengen im Vakuum isoliert. Die Reinigung erfolgt nach chromatographischen Methoden.

Um Verbindungen der Formel VIII

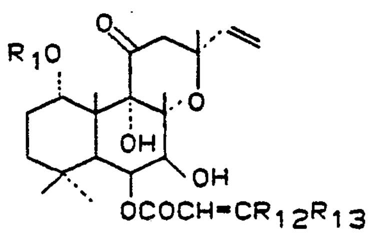

VIII

worin $R_1$ und $R_{12}$ die obigen Bedeutungen haben, zu erhalten, behandelt man Verbindungen der Formel VII weiterhin mit Alkali wie Natriumhydroxid in wasserlöslichen organischen Lösungsmitteln wie Acetonitril.

Verbindungen der Formel VIII werden weiterhin mit Halogenalkylformiat in Gegenwart von Pyridin und 4-Dimethylaminopyridin in organischen Lösungsmitteln wie Dichlormethan oder Essigester behandelt, wobei Verbindungen der Formel IX erhalten werden, worin A für $OR_2$ steht und $R_2$ sowie $R_1$, $R_{12}$ und $R_{13}$ die obigen Bedeutungen haben, oder mit dem entsprechenden Carbamoylchlorid in Gegenwart von 4-Dimethylaminopyridin und Hydrochinonmonoethylether in einem organischen Lösungsmittel wie Pyridin, was Verbindungen der Formel IX

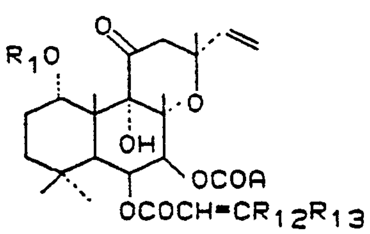

IX

ergibt, worin A für

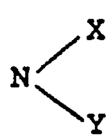

steht, wobei X und Y sowie $R_1$, $R_{12}$ und $R_{13}$ die obigen Bedeutungen haben.

Verbindungen der Formel IX behandelt man mit dem entsprechenden Amin der Formel HNX·Y· worin

$X_1$ bzw. $Y_1$ die oben angegebenen Bedeutungen haben, in einem organischen Lösungsmittel wie Dichlormethan 16 bis 24 Stunden lang bei 20 bis 30 °C. Das Produkt gewinnt man aus dem Reaktionsgemisch durch Extrahieren mit organischem Lösungsmittel, Waschen des Extrakts mit Wasser, Trocknen über wasserfreiem Natriumsulfat und Einengen im Vakuum. Die Verbindungen der Formel I werden durch Säulenchromatographie gereinigt.

Die erfindungsgemäßen Verbindungen und deren Salze weisen die der Klasse der mehrfach oxydierten Labdane und deren Derivaten zugeschriebenen pharmakologischen Eigenschaften auf. Sie zeigen aber insbesondere selektiv positiv inotrope Wirkung, blutdrucksenkende Wirkung und eine Senkung des intraokularen Drucks. Dies wird durch die nachfolgenden pharmakologischen Untersuchungen, die zur Auswertung der erfindungsgemäßen Verbindungen und deren Salze durchgeführt wurden, und die dabei erhaltenen Ergebnisse veranschaulicht.

**Positive inotrope Wirksamkeit**

Dafür wurde folgende Methode angewandt.

400 g schwere Meerschweinchen beider Geschlechter werden getötet, das Herz entnommen und bei Raumtemperatur in Ringer-Lösung eingebracht. Sowohl der linke als auch der rechte Vorhof werden dann isoliert, an einem Organhalter befestigt und in eine Ringer-Lösung enthaltendes, bei einer Temperatur von 32 °C gehaltenes Bad eingebracht. In das Organbad läßt man ein Gemisch von 95 % $O_2$ und 5 % $CO_2$ einperlen. Dann stimuliert man den Vorhof elektrisch. Die zu prüfende erfindungsgemäße Verbindung wird in Wasser zu einer Lösung bekannter Konzentration gelöst und dem Bad zugesetzt. Die Kontraktionskraft des Vorhofs wird 7 bis 10 Minuten lang über einen isometrischen Dehnungsmeßstreifen auf einem 4-Kanal-Schreiber von Nihon Kohden registriert. Die Aktivität wird gemäß den erhaltenen Daten als $EC_{50}$ ausgedrückt.

Die bei diesem Modell für repräsentative erfindungsgemäße Verbindungen erhaltenen Ergebnisse sind in der nachfolgenden Tabelle aufgeführt.

| Verbindung | | Meerschweinchenvorhof $EC_{50}$ g/ml |
|---|---|---|
| 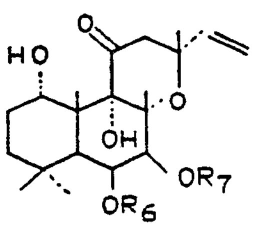 •HCl | | |
| $R_6$ | $R_7$ | |
| $CO(CH_2)_2N$ 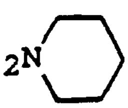 | $COOC_2H_5$ | 0 |
| $CO(CH_2)_2N$ 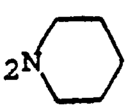 | $CON(C_2H_5)_2$ | 0,79 |
| $COCH_2N$ 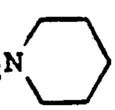 | $COOC_2H_5$ | 1,6 |
| $COCH_2N$ 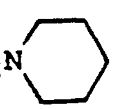 | $CON(C_2H_5)_2$ | 0,44 |

## Intraokulare Druckmessung

### Messung des Augeninnen-Drucks am wachen Kaninchen

Für diesen Versuch werden 2 bis 3 kg schwere Kaninchen beider Geschlechter verwendet. Der intraokulare Druck (IOD) wird mit einem Schioetz-Tonometer nach Hornhaut-Anästhesie mit 2 %iger Novocainlösung gemessen. Zur Herstellung einer 2 %igen Lösung wird eine erfindungsgemäße Verbindung unter Verwendung der stöchiometrischen Menge 0,1 n-HCl, bzw. deren Salz direkt in Wasser gelöst. Nach Ermittlung des Ausgangswertes werden 100 µl der Lösung der Testverbindung in eines der Augen, das Vehikel in das andere Auge instilliert. Der IOD wird in bestimmten Zeitabständen, d.h. 0,5; 1, 2, 3, 4 und 5 Stunden gemessen. Die prozentuale Abnahme des IOD wird anhand des Ausgangswertes berechnet.

Die bei diesem Modell für repräsentative erfindungsgemäße Verbindungen erhaltenen Ergebnisse sind in der nachfolgenden Tabelle aufgeführt:

**Verbindung** | **IOD-senkende Wirkung**

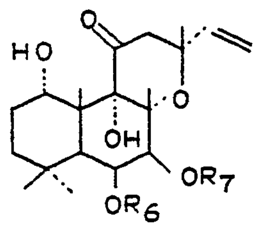 •HCl

| $R_6$ | $R_7$ | Dosis Prozent | % Abnahme des IOD | Dauer (min) |
|---|---|---|---|---|
| 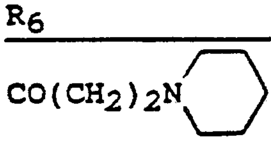 | $COOC_2H_5$ | 2 | 20 | 300 |
| 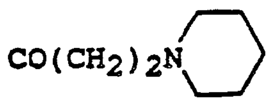 | $CON(C_2H_5)_2$ | 2 | 25 | 360 |
| 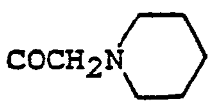 | $COOC_2H_5$ | 2 | 0 | - |
| 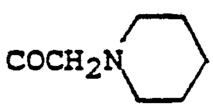 | $CON(C_2H_5)_2$ | 2 | 20 | 420 |

## Bestimmung der Blutdrucksenkung

### Blutdruck an der Katze:

3 bis 4 kg schwere Katzen beider Geschlechter werden mit Äther anästhesiert und unter Chloralose-Narkose (70 mg/kg i.v.) gehalten. Sowohl die Oberschenkel-Arterie als auch die -Vene werden zur Aufzeichnung des Blutdrucks bzw. zur Verabreichung der Arzneimittel mit Kanülen versehen. Der Blutdruck in der Oberschenkelarterie wird über einen Druckaufnehmer Statham P 23 Db auf einem Nihon-Kohden-Schreiber für physiologische Zwecke ausgezeichnet. Die zu prüfende Verbindung wird in destilliertem

Wasser gelöst und intravenös verabreicht. Der Blutdruckabfall und die Dauer der blutdrucksenkenden Wirkung werden notiert.

Die bei diesem Modell für repräsentative erfindungsgemäße Verbindungen erhaltenen Ergebnisse sind in der nachfolgenden Tabelle aufgeführt:

**Verbindung**

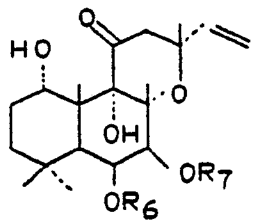

•HCl

| | | Dosis (mg/kg) | Abfall d. BD | Dauer (min) |
|---|---|---|---|---|
| $CO(CH_2)_2N$ 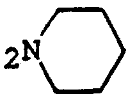 | $COOC_2H_5$ | 1 | 40 | >20 |
| $CO(CH_2)_2N$ 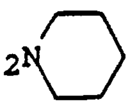 | $CON(C_2H_5)_2$ | 1 | 20 | 60 |
| $COCH_2N$ 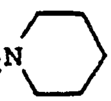 | $COOC_2H_5$ | 3 | 60 | 60 |
| $COCH_2N$ 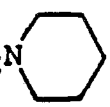 | $CON(C_2H_5)_2$ | 1 | 50 | 20 |

Die Erfindung wird durch die nachfolgenden Beispiele veranschaulicht:

**Beispiel 1**

**1 α-t-Butyldimethylsilyloxy-7β-crotonyloxy-6β,9α-dihydroxy-8,13-epoxy-labd-14-en-11-on**

Unter Rühren gibt man Dicyclohexylcarbodiimid (2,5 g, 12,14 mmol) zu einem Gemisch aus 4-N-Dimethylaminopyridin (0,45 g, 3,68 mmol), Crotonsäure (1,0 g, 11,62 mmol) und 1α-t-Butyldimethylsilyloxy-8,13-epoxy-6β,7β,9α-trihydroxy-labd-14-en-11-on (4,0 g, 8,30 mmol) in trockenem DMF (10 ml). Man rührt das Reaktionsgemisch vier Stunden lang, gießt auf Eis und extrahiert mit Essigester. Die organische Schicht wird mit Wasser und dann Kochsalzlösung gewaschen, über wasserfreiem Natriumsulfat getrocknet und eingeengt. Den Rückstand reinigt man durch Flash-Säulenchromatographie mit Essigester : Petrolether (1 : 9) als Eluiermittel; Schmelzpunkt 114-16°, Ausbeute 64 %.

In entsprechender Weise wird die Verbindung, 7β-Acryloyloxy-1α-t-butyldimethylsilyloxy-6β,9α-dihydroxy-8,13-epoxy-labd-14-en-11-on hergestellt.

**Beispiel 2**

**1 α-t-Butyldimethylsilyloxy-6β-crotonyloxy-7β,9α-dihydroxy-8,13-epoxy-labd-14-en-11-on**

Unter Rühren gibt man Natronlauge (9,6 ml, 1 n) zu 1α-t-Butyldimethylsilyloxy-7β-crotonyloxy-6β,9α-dihydroxy-8,13-epoxy-labd-14-en-11-on (2,8 g, 5,09 mmol) in Acetonitril: Wasser (1 : 1, 320 ml), rührt noch 45 Minuten lang, engt im Vakuum ein und extrahiert mit Essigester. Die organische Schicht wird mit Wasser und danach mit Kochsalzlösung gewaschen, über wasserfreiem Natriumsulfat getrocknet und eingeengt. Den Rückstand reinigt man durch Flash-Säulenchromatographie mit Essigester : Petrolether (15 : 85) als Eluiermittel. Schmelzpunkt 85-87° C, Ausbeute 71,2 %.

Die Verbindung 6β-Acryloyloxy-1α-t-butyldimethylsilyloxy-7β,9α-dihydroxy-8,13-epoxy-labd-14-en-11-on (Schmelzpunkt 164-166° C) wird auf ähnliche Weise hergestellt.

**Beispiel 3**

**1 α-t-Butyldimethylsilyloxy-6β-crotonyloxy-7β-ethoxycarbonyloxy-8,13-epoxy-9α-hydroxy-labd-14-en-11-on**

Unter Rühren gibt man Chlorameisensäureethylester (2,5 ml, 26,14 mmol) zu einem Gemisch aus 4-N-Dimethylaminopyridin (0,1 g, 0,81 mmol), Pyridin (2,5 ml) und 1α-t-Butyldimethylsilyloxy-6β-crotonyloxy-7β,9α-dihydroxy-8,13-epoxy-labd-14-en-11-on (0,8 g, 1,45 mmol) in Dichlormethan (15 ml). Man rührt über Nacht und behandelt mit einer weiteren Menge Chlorameisensäureethylester (2,0 ml, 20,91 mmol) und Pyridin (2 ml). Man rührt noch 12 Stunden weiter, erhitzt dann 4 Stunden lang auf 60 bis 70° C, gießt auf Eis und extrahiert mit Essigester. Die organische Schicht wird mit verdünnter HCl, Wasser und danach Kochsalzlösung gewaschen, über wasserfreiem Natriumsulfat getrocknet und eingeengt. Schmelzpunkt 116-18° C.

Die folgenden Verbindungen werden auf ähnliche Weise synthetisiert:

1. 6β-Acryloyloxy-1α-t-butyldimethylsilyloxy-7β-ethoxycarbonyloxy-8,13-epoxy-9α-hydroxy-labd-14-en-11-on

2 1α-t-Butyldimethylsilyloxy-7β-ethoxycarbonyloxy-8,13-epoxy-9α-hydroxy-6β-piperidinoacetoxy-labd-14-en-11-on

**Beispiel 4**

**6β-Acryloyloxy-1α,9α-dihydroxy-7β-ethoxycarbonyloxy-8,13-epoxy-labd-14-en-11-on**

Man gibt Tetrabutylammoniumfluorid-trihydrat (0,8 g, 2,54 mmol) zu einer Lösung von 6β-Acryloyl-1α-t-butyldimethylsilyloxy-7β-ethoxycarbonyloxy-8,13-epoxy-9α-hydroxy-labd-14-en-11-on (1,4 g, 2,3 mmol) in THF (60 ml), rührt 10 Minuten lang bei Raumtemperatur und engt im Vakuum ein. Der Rückstand wird mit Essigester extrahiert und die organische Schicht mit Wasser gewaschen, über wasserfreiem Natriumsulfat getrocknet und eingeengt. Den Rückstand reinigt man durch Flash-Säulenchromatographie mit Acetonitril : Chloroform : Diisopropylether : Petrolether (7 : 25 : 25 : 18), Ausbeute 92,3 %, Öl.

Die folgenden Verbindungen werden auf ähnliche Weise synhetisiert:

1. 6β-Crotonyloxy-1α,9α-dihydroxy-7β-ethoxycarbonyloxy-8,13-epoxy-labd-14-en-11-on, Schmelzpunkt 88-90° C.
2. 6β-Crotonyloxy-7β-N,N-diethylaminocarbonyloxy-1α,9α-dihydroxy-8,13-epoxy-labd-14-en-11-on, Schmelzpunkt 76-79° C.
3. 6β-Acryloyloxy-7β-N,N-diethylaminocarbonyloxy-1α,9α-dihydroxy-8,13-epoxy-labd-14-en-11-on, Schmelzpunkt 168-70° C.
4. 1α,9α-Dihydroxy-7β-ethoxycarbonyl-8,13-epoxy-6β-piperidinoacetoxy-labd-14-en-11-on.
5. 7β-N,N-Diethylaminocarbonyloxy-1α,9α-dihydroxy-8,13-epoxy-6β-piperidinoacetoxy-labd-14-en-11-on.

6. 7β-Anilinocarbonyloxy-1α,9α-dihydroxy-8,13-epoxy-6β-morpholinoacetoxy-labd-14-en-11-on.

**Beispiel 5**

**6β-Acryloyloxy-1α-t-butyldimethylsilyloxy-7β-N,N-diethylaminocarbonyloxy-8,13-epoxy-9α-hydroxy-labd-14-en-11-on**

Unter Rühren gibt man N,N-Diethylcarbamoylchlorid (3,0 ml, 23,67 mmol) zu einem Gemisch aus 4-N-Dimethylaminopyridin (0,1 g, 0,81 mmol), Hydrochinonmonomethylether (0,05 g, 0,4 mmol) und 6β-Acryloyloxy-1α-t-butyldimethylsilyloxy-7β,9α-dihydroxy-8,13-epoxy-labd-14-en-11-on (1,5 g, 2,79 mmol) in Pyridin (10 ml), erhitzt 16 Stunden lang am Rückfluß, gibt eine weitere Menge N,N-Diethylcarbamoylchlorid (2 ml, 5,75 mmol) dazu, erhitzt für weitere drei Stunden am Rückfluß und engt im Vakuum ein. Der Rückstand wird mit Essigester extrahiert und die organische Schicht mit verdünnter HCl, Wasser und danach Kochsalzlösung gewaschen, über wasserfreiem Natriumsulfat getrocknet und eingeengt. Den Rückstand reinigt man durch Flash-Säulenchromatographie mit Chloroform : Diisopropylether : Petrolether (1 : 1 : 2), Ausbeute 90 %.

Die folgenden Verbindungen werden auf ähnliche Weise synthetisiert:

1. 1α-t-Butyldimethylsilyloxy-7β-N,N-diethylaminocarbonyloxy-8,13-epoxy-9α-hydroxy-6β-piperidinoacetoxy-labd-14-en-11-on.

2. 1α-t-Butyldimethylsilyloxy-6β-crotonyloxy-7β-N,N, diethylaminocarbonoxy-8,13-epoxy-9α-hydroxy-labd-14-en-11-on.

**Beispiel 6**

**7β-N,N-Diethylaminocarbonyloxy-1α,9α-dihydroxy-8,13-epoxy-6β-(3′-morpholinopropionyloxy)-labd-14-en-11-on**

Unter Rühren gibt man Morpholin (1,5 ml, 17,2 mmol) zu einer Lösung von 6β-Acryloyloxy-7β-N,N-diethylaminocarbonyloxy-1α,9α-dihydroxy-8,13-epoxy-labd-14-en-11-on (0,42 g, 0,80 mmol) in Dichlormethan (15 ml), rührt noch weitere 16 Stunden bei Raumtemperatur und engt ein. Man extrahiert den Rückstand mit Essigester, wäscht mit Wasser und Kochsalzlösung, trocknet über wasserfreiem Natriumsulfat und engt im Vakuum ein. Den Rückstand reinigt man durch Flash-Säulenchromatographie mit Essigester : Petrolether : Triethylamin (65 : 35 : 1) als Eluiermittel. Schmelzpunkt 180-82 °C, aus Chloroform/Petrolether umkristallisiert.

Die folgenden Verbindungen werden auf ähnliche Weise hergestellt:

1. 7β-N,N-Diethylaminocarbonyloxy-1α,9α-dihydroxy-8,13-epoxy-6β-(3-piperidinobutyryloxy)-labd-14-en-11-on.

2. 1α,9α-Dihydroxy-7β-ethoxycarbonyloxy-8,13-epoxy-6β-(3′-piperidinobutyryloxy)-labd-14-en-11-on.

3. 1α,9α-Dihydroxy-7β-ethoxycarbonyloxy-8,13-epoxy-6β-(3′-morpholinopropionyloxy)-labd-14-en-11-on.

4. 1α,9α-Dihydroxy-6β-(3′-N,N-dimethylaminopropyionyloxy)-7β-ethoxycarbonyloxy-8,13-epoxy-labd-14-en-11-on.

5. 7β-N,N-Diethylamino-1α,9α-dihydroxy-6β-(3′-dimethylaminopropionyloxy)-8,13-epoxy-labd-14-en-11-on.

**Beispiel 7**

**7β-N,N-Diethylaminocarbonyloxy-1α,9α-dihydroxy-8,13-epoxy-6β-(3′-morpholinopropionyloxy)-labd-14-en-11-on-hydrochlorid-hemihydrat**

Man gibt Diethylether (10 ml, bei 0° C mit trockenem HCl-Gas gesättigt) zu einer methanolischen Lösung von 7β-N,N-Diethylaminocarbonyloxy-1α,9α-dihydroxy-8,13-epoxy-6β-(3′-morpholinopropionyloxy)-labd-14-en-11-on (0,3 g in 3 ml Methanol), verdünnt mit überschüssigem trockenen Diethylether und trennt den ausgeschiedenen Niederschlag durch Filtrieren ab. Umkristallisieren des Feststoffs aus Methanol/Ether liefert 7β-N,N-Diethylaminocarbonyloxy-1α,9α-dihydroxy-8,13-epoxy-6β-(3′-morpholinopropionyloxy)-labd-14-en-11-on-hydrochlorid-hemihydrat, Ausbeute 95 %, Schmelzpunkt 160-64° C.

Die folgenden Verbindungen werden auf ähnliche Weise synthetisiert:

1. 7β-N,N-Diethylaminocarbonyloxy-1α,9α-dihydroxy-6β-(3′-N,N-dimethylaminopropionyloxy)-8,13-epoxy-labd-14-en-11-on-hydrochlorid, Schmelzpunkt 239-240° C.
2. 7β-N,N-Diethylaminocarbonyloxy-1α,9α-dihydroxy-8,13-epoxy-6β-(3′-piperidinopropionyl)-labd-14-en-11-on-hydrochlorid, Schmelzpunkt 246-247° C.
3. 7β-N,N-Diethylaminocarbonyloxy-1α,9α-dihydroxy-8,13-epoxy-6β-(3′-piperidinobutyryloxy)-labd-14-en-11-on-hydrochlorid, Schmelzpunkt 173-175° C.
4. 7β-N,N-Diethylaminocarbonyloxy-1α,9α-dihydroxy-8,13-epoxy-6β-piperidinoacetoxy-labd-14-en-11-on-hydrochlorid, Schmelzpunkt 253° C.
5. 6β-(3′-N,N-Dimethylaminopropionyloxy)-1α,9α-dihydroxy-7β-ethoxycarbonyloxy-8,13-epoxy-labd-14-en-11-on-hydrochlorid-hemihydrat, Schmelzpunkt 213-215° C.
6. 1α,9α-Dihydroxy-7β-ethoxycarbonyloxy-8,13-epoxy-6β-(3′-piperidinopropionyloxy)-labd-14-en-11-on-hydrochlorid, Schmelzpunkt 227-228° C.
7. 1α,9α-Dihydroxy-7β-ethoxycarbonyloxy-8,13-epoxy-6β-(3′-morpholinopropionyloxy)-labd-14-en-11-on-hydrochlorid, Schmelzpunkt 218-220° C.
8. 1α,9α-Dihydroxy-7β-ethoxycarbonyloxy-8,13-epoxy-6β-(3′-piperidinobutyryloxy)-labd-14-en-11-on-hydrochlorid, Schmelzpunkt 175-178° C.
9. 1α,9α-Dihydroxy-7β-ethoxycarbonyloxy-8,13-epoxy-6β-piperidinoacetoxy-labd-14-en-11-on-hydrochlorid, Schmelzpunkt 268° C.
10. 7β-Anilinocarbonyloxy-1α,9α-dihydroxy-8,13-epoxy-6β-morpholinoacetoxy-labd-14-en-11-on-hydrochlorid-sesquihydrat, Schmelzpunkt 201 - 205° C.

## Beispiel 8

### 7β-Anilinocarbonyloxy-1α-t-butyldimethyl-silyloxy-8,13-epoxy-9α-hydroxy-6β-morpholino-acetoxy-labd-14-en-11-on

0,075 $m^2$ Phenylisocynat (0,69 mmol) wurde zu einer gerü+hrten Mischung von 0,385 g 1α-t-Butyldimethylsilyloxy-7β,9α-dihydroxy-8,13-epoxy-6β-morpholino-acetoxy-labd-14-en-11-on (0,632 mmol) und 0,096 $m^2$ Triethylamin (0,688 mmol) in 10 $m^2$ Toluol hinzugegen. Die Reaktionsmischung wurde 17 Stunden lang am Rückfluß erhitzt. Eine weitere Menge von 0,3 $m^2$ Phenylisocyanat (2,76 mmol) wurde zur Reaktionsmischuhinzugefügt und weitere 3 Stunden am Rückfluß erhitzt. Die Reaktionsmischung wurde dann im Vakuum eingeengt und mit Chloroform extrahiert. Die organische Schicht wurde mit Wasser und gesättigter Kochsalzlösung gewaschen, über Natriumsulfat getrocknet und eingeengt. Der Rückstand ergab nach Reinigung durch Flash-Chromatographie mit Essigester:Petrolether:Acetonitril (6:13:1) als Eluiermittel das reine Produkt als dickes Öl. Ausbeute 90 %.

## Ansprüche

1. Verbindungen der Formel I

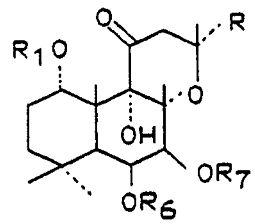

I

worin bedeuten
R Vinyl, Ethyl, Cyclopropyl oder $CHOHCH_2OH$,
$R_1$ Wasserstoff, eine Gruppe der Formel
$-\overset{\overset{O}{\|}}{C}-A$, wobei A $OR_2$, worin $R_2$ eine Alkylgruppe darstellt, oder

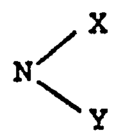

bedeutet,
wobei X und Y, falls sie gleich sind, Wasserstoff oder Alkyl darstellen, oder, falls X für Wasserstoff oder Niederalkyl steht, Y eine Alkyl-, substituierte Alkyl-, Cycloalkyl-, Aralkyl-, Aryl-, Amino- oder Hydroxylgruppe darstellt, oder X und Y mit dem Stickstoffatom, an das sie gebunden sind, einen heterocyclischen Ring bilden, der ein weiteres Heteroatom enthalten und durch eine Alkyl- oder Arylgruppe substituiert sein kann, oder für eine Gruppe der Formel $R_3R_4R_5Si$, wobei $R_3$, $R_4$ und $R_5$ unabhängig je eine Alkylgruppe bedeuten,
$R_6$ eine Gruppe der Formel

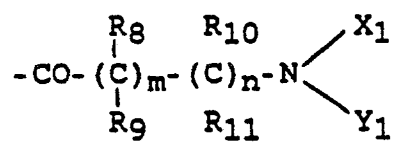

worin m und n ganze Zahlen von 0 bis 10 sind und $R_8$ und $R_9$ gleich oder verschieden sind und Wasserstoff oder eine Niederalkylgruppe darstellen, oder einer der Substituenten Wasserstoff und der andere eine Hydroxy-, Thio-, oder Arylgruppe darstellt, $R_{10}$ Wasserstoff und $R_{11}$ Wasserstoff oder eine Hydroxy- oder Alkylgruppe bedeuten, und $X_1$ Wasserstoff darstellt, falls $Y_1$ für Wasserstoff, Alkyl, substituiertes Alkyl, Alkanoyl, Aryl, Cycloalkyl, Aralkyl, einen Heterocyclus, Amino, substituiertes Amino, Hydroxy, Acryl, Dialkylaminoalkyl, Carbamoyl, Carboxyalkyl oder Carbalkoxyalkyl steht, oder $X_1$ und $Y_1$, falls sie gleich sind, Alkyl, substituiertes Alkyl, Aryl oder Aralkyl darstellen oder, falls $X_1$ für Alkyl steht, $Y_1$ substituiertes Alkyl, Cycloalkyl, Aralkyl oder eine Dialkylaminoalkylgruppe darstellt, oder $X_1$ und $Y_1$ zusammen mit dem Stickstoffatom, an das sie gebunden sind, einen Heterocyclus bilden, der ein oder mehrere Heteroatome enthalten und gegebenenfalls ein- oder mehrfach durch Alkyl-, Aryl-, Aralkyl-, Hydroxyalkyl-, Hydroxyl- oder andere heterocyclische Gruppen substituiert sein kann,
$R_7$ eine Gruppe der Formel
$-\overset{\overset{O}{\|}}{C}-A$, worin A dieselbe Bedeutung wie oben für $R_1$ oder
$-\overset{\overset{O}{\|}}{C}-A$ angegeben hat, oder A eine Alkylgruppe bedeutet, wenn $R_6$ für eine Gruppe der Formel

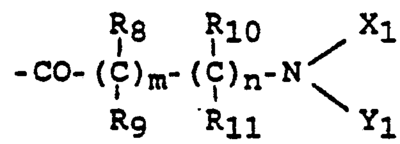

steht, worin $R_8$ bis $R_{11}$, $X_1$, $Y_1$, m und n die obigen Bedeutungen haben, sowie deren optische und geometrische Isomere und deren pharmazeutisch unbedenkliche Säureadditionssalze.

2. Verbindungen der Formel I gemäß Anspruch 1, worin R die Vinylgruppe, $R_1$ Wasserstoff und $R_6$ und $R_7$ die angegebenen Bedeutungen haben, deren optische und geometrische Isomere sowie pharmazeutisch unbedenkliche Säureadditionssalze.

3. Verbindungen gemäß der Formel I gemäß Anspruch 2, worin $R_6$ eine Gruppe der Formel

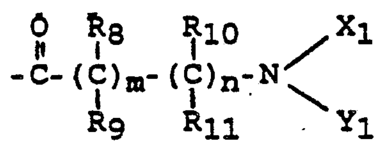

bedeutet, worin $R_8$, $R_9$, $R_{10}$ und $R_{11}$ gleich oder verschieden sind und jeweils Wasserstoff oder Methyl bedeuten, die Summe von m und n eine ganze Zahl von 1 bis 5 beträgt, $X_1$ und $Y_1$ entweder gleich sind und Wasserstoff oder $C_1$-$C_4$-Alkyl bedeuten, oder einer der Substituenten Wasserstoff und der andere eine $C_1$-$C_4$-Alkylgruppe bedeutet, oder $X_1$ und $Y_1$ zusammen mit dem Stickstoffatom, an das sie gebunden sind, einen gegebenenfalls substituierten Piperidino-, Pyrrolidino-, Morpholino-, Piperazino-, Thiomorpholino-, Imidazolino-oder Theophyllino-Rest darstellen, und $R_7$ den Rest

$-\overset{\overset{O}{\|}}{C}$ -A bedeutet, worin A eine $C_1$-$C_4$-Alkoxygruppe oder eine Gruppe der Formel

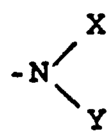

darstellt, worin X und Y die genannten Bedeutungen haben, deren optische und geometrische Isomere sowie pharmazeutisch unbedenkliche Säureadditionssalze.

4. Verbindungen der Formel I gemäß Anspruch 3, worin $R_6$ eine Gruppe der Formel

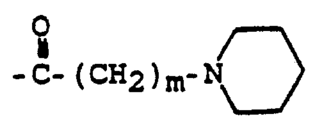

worin m die Zahl 1 oder 2 bedeutet und $R_7$ den Rest

$-\overset{\overset{O}{\|}}{C}$ -A darstellt, worin A die Ethoxy- oder Diethylaminogruppe bedeutet, deren optische und geometrische Isomere sowie pharmazeutisch unbedenklichen Säureadditionssalze.

5. Verfahren zur Herstellung von Verbindungen der Formel I gemäß Anspruch 1, dadurch gekennzeichnet, daß man eine Verbindung der Formel II

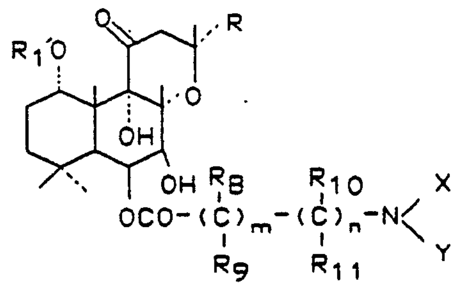

II

worin $R_1'$ eine Schutzgruppe für eine alkoholische Hydroxylgruppe ist und R, $R_8$ bis $R_{11}$ sowie $X_1$ und $Y_2$ die genannten Bedeutungen haben, entweder

a) mit einem Gemisch eines Halogenalkylformiats und 4-Dimethylaminopyridin umsetzt zu einer Verbindung der Formel III,

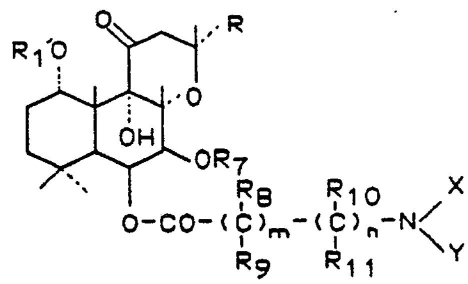

III

worin $R_7$ einen Rest der Formel

$-\overset{O}{\overset{\|}{C}}-OR_2$ mit $R_2$ $C_1$-$C_8$-Alkyl darstellt, oder

b) mit einem Gemisch aus einer Verbindung der Formel

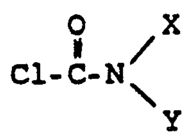

und 4-Dimethylaminopyridin und Hydrochinonmonomethylether umsetzt, zu einer Verbindung der Formel III, worin $R_7$ einen Rest der Formel

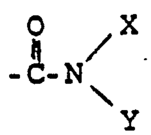

darstellt, worin X und Y die genannten Bedeutungen haben,

c) aus einer Verbindung der Formel III nach üblichen Methoden die Schutzgruppe $R_1'$ abspaltet, wodurch man eine Verbindung der Formel I erhält, worin $R_1$ Wasserstoff bedeutet, oder

d) eine Verbindung der Formel IX

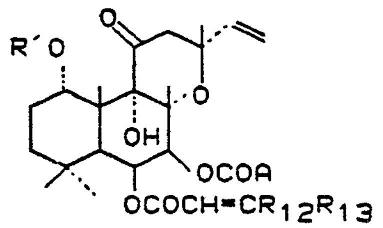

IX

worin $R_1'$ eine Schutzgruppe, A die obengenannte Bedeutung hat und $R_{12}$ und $R_{13}$ jeweils Wasserstoff oder eine Niederalkyl- oder Arylgruppe darstellen, mit einem Amin dere Formel

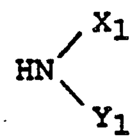

umsetzt, worin $X_1$ und $Y_1$ die genannten Bedeutungen haben, und anschließend nach üblichen Methoden die Schutzgruppe $R_1'$ abspaltet, wodurch eine Verbindung der Formel I worin $R_1$ Wassertoff ist, erhalten wird.

6. Arzneimittel, gekennzeichnet durch einen Gehalt an einer Verbindung gemäß Anspruch 1 und üblichen Hilfs- und Trägerstoffen.

7. Verwendung einer Verbindung gemäß Anspruch 1 zur Herstellung eines Arzneimittels mit positiv inotroper Augeninnendruck-senkender oder blutdrucksenkender Wirkung.

Patentansprüche für folgende Vertragsstaaten: ES, GR

1. Verfahren zur Herstellung von Verbindungen der Formel I

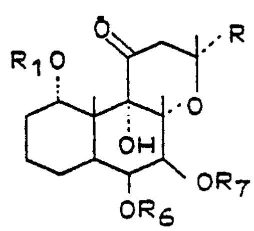

I

worin bedeuten

R Vinyl, Ethyl, Cyclopropyl oder $CHOHCH_2OH$, $R_1$ Wasserstoff, eine Gruppe der Formel $-\overset{O}{\overset{\|}{C}}-A$, wobei A $OR_2$, worin $R_2$ eine Alkylgruppe darstellt, oder

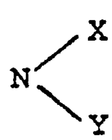

bedeutet,

wobei X und Y, falls sie gleich sind, Wasserstoff oder Alkyl darstellen, oder, falls X für Wasserstoff oder Niederalkyl steht, Y eine Alkyl-, substituierte Alkyl-, Cycloalkyl-, Aralkyl-, Aryl-, Amino- oder Hydroxylgruppe darstellt, oder X und Y mit dem Stickstoffatom, an das sie gebunden sind, einen heterocyclischen Ring bilden, der ein weiteres Heteroatom enthalten und durch eine Alkyl- oder Arylgruppe substituiert sein kann, oder für eine Gruppe der Formel $R_3R_4R_5Si$, wobei $R_3$, $R_4$ und $R_5$ unabhängig je eine Alkylgruppe bedeuten,

$R_6$ eine Gruppe der Formel

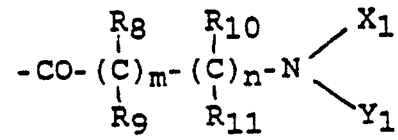

worin m und n ganze Zahlen von 0 bis 10 sind und $R_8$ und $R_9$ gleich oder verschieden sind und Wasserstoff oder eine Niederalkylgruppe darstellen, oder einer der Substituenten Wasserstoff und der andere eine Hydroxy-, Thio-, oder Arylgruppe darstellt, $R_{10}$ Wasserstoff und $R_{11}$ Wasserstoff oder eine Hydroxy- oder Alkylgruppe bedeuten, und $X_1$ Wasserstoff darstellt, falls $Y_1$ für Wasserstoff, Alkyl, substituiertes Alkyl, Alkanoyl, Aryl, Cycloalkyl, Aralkyl, einen Heterocyclus, Amino, substituiertes Amino, Hydroxy, Acryl, Dialkylaminoalkyl, Carbamoyl, Carboxyalkyl oder Carbalkoxyalkyl steht, oder $X_1$ und $Y_1$, falls sie gleich sind, Alkyl, substituiertes Alkyl, Aryl oder Aralkyl darstellen oder, falls $X_1$ für Alkyl steht, $Y_1$ substituiertes Alkyl, Cycloalkyl, Aralkyl oder eine Dialkylaminoalkylgruppe darstellt, oder $X_1$ und $Y_1$ zusammen mit dem Stickstoffatom, an das sie gebunden sind, einen Heterocyclus bilden, der ein oder mehrere Heteroatome enthalten und gegebenenfalls ein- oder mehrfach durch Alkyl-, Aryl-, Aralkyl-, Hydroxyalkyl-, Hydroxyl- oder andere heterocyclische Gruppen substituiert sein kann,

$R_7$ eine Gruppe der Formel

$-\overset{O}{\overset{||}{C}}-A$, worin A dieselbe Bedeutung wie oben für $R_1$ oder

$-\overset{O}{\overset{||}{C}}-A$ angegeben hat, oder A eine Alkylgruppe bedeutet, wenn $R_6$ für eine Gruppe der Formel

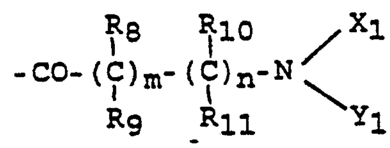

steht, worin $R_8$ bis $R_{11}$, $X_1$, $Y_1$, m und n die obigen Bedeutungen haben, sowie deren optischenund geometrischen Isomeren und deren pharmazeutisch unbedenklichen Säureadditionssalzen, dadurch gekennzeichnet, daß man eine Verbindung der Formel II

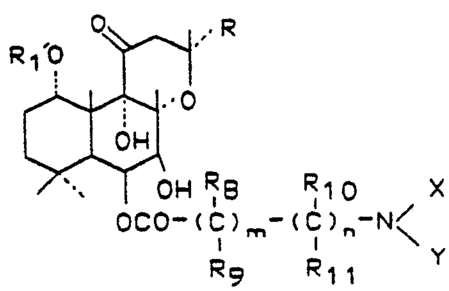

II

worin $R_1'$ eine Schutzgruppe für eine alkoholische Hydroxylgruppe ist und R, $R_8$ bis $R_{11}$ sowie $X_1$ und $Y_2$ die genannten Bedeutungen haben, entweder

a) mit einem Gemisch eines Halogenalkylformiats und 4-Dimethylaminopyridin umsetzt zu einer Verbindung der Formel III,

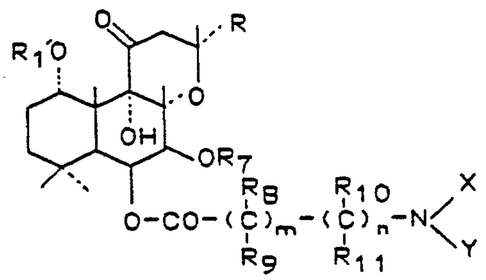

III

worin $R_7$ einen Rest der Formel

$-\overset{\overset{O}{\|}}{C}-OR_2$ mit $R_2$ $C_1$-$C_8$-Alkyl darstellt, oder

b) mit einem Gemisch aus einer Verbindung der Formel

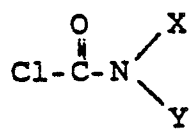

und 4-Dimethylaminopyridin und Hydrochinonmonomethylether umsetzt, zu einer Verbindung der Formel III, worin $R_7$ einen Rest der Formel

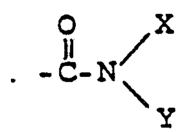

darstellt, worin X und Y die genannten Bedeutungen haben,

c) aus einer Verbindung der Formel III nach üblichen Methoden die Schutzgruppe $R_1'$ abspaltet, wodurch man eine Verbindung der Formel I erhält, worin $R_1$ Wasserstoff bedeutet, oder

d) eine Verbindung der Formel IX

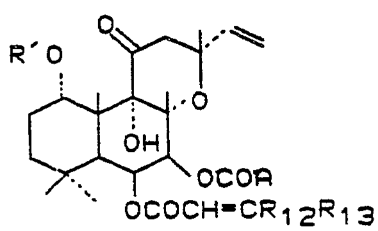

IX

worin $R_1'$ eine Schutzgruppe, A die obengenannte Bedeutung hat und $R_{12}$ und $R_{13}$ jeweils Wasserstoff oder eine Niederalkyl- oder Arylgruppe darstellen, mit einem Amin der Formel

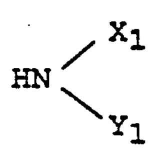

umsetzt, worin $X_1$ und $Y_1$ die genannten Bedeutungen haben, und anschließend nach üblichen Methoden die Schutzgruppe $R_1'$ abspaltet, wodurch eine Verbindung der Formel I worin $R_1$ Wasserstoff ist, erhalten wird.

2. Verfahren gemäß Anspruch 1, dadurch gekennzeichnet, daß in den in Anspruch 1 genannten Verbindungen R für die Vinylgruppe, $R_1$ für Wasserstoff steht und $R_6$ und $R_7$ die angegebenen Bedeutungen haben.

3. Verfahren gemäß Anspruch 2, dadurch gekennzeichnet, daß in den in Anspruch 1 genannten Verbindungen $R_6$ eine Gruppe der Formel

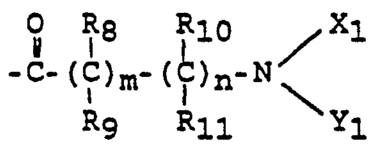

bedeutet, worin $R_8$, $R_9$, $R_{10}$ und $R_{11}$ gleich oder verschieden sind und jeweils Wasserstoff oder Methyl bedeuten, die Summe von m und n eine ganze Zahl von 1 bis 5 beträgt, $X_1$ und $Y_1$ entweder gleich sind und Wasserstoff oder $C_1$-$C_4$-Alkyl bedeuten, oder einer der Substituenten Wasserstoff und der andere eine $C_1$-$C_4$-Alkylgruppe bedeutet, oder $X_1$ und $Y_1$ zusammen mit dem Stickstoffatom, an das sie gebunden sind, einen gegebenenfalls substituierten Piperidino-, Pyrrolidino-, Morpholino, Piperazino-, Thiomorpholino-, Imidazolino-oder Theophyllino-Rest darstellen, und $R_7$ den Rest

$-\overset{O}{\overset{\|}{C}}-A$ bedeutet, worin A eine $C_1$-$C_4$-Alkoxygruppe oder eine Gruppe der Formel

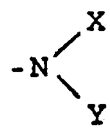

darstellt, worin X und Y die genannten Bedeutungen haben.

4. Verfahren gemäß Anspruch 3, dadurch gekennzeichnet, daß in den in Anspruch 1 genannten Verbindungen der Formel I $R_6$ für eine Gruppe der Formel

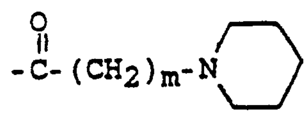

steht worin m die Zahl 1 oder 2 bedeutet und $R_7$ den Rest

$-\overset{O}{\overset{\|}{C}}-A$ darstellt, worin A die Ethoxy- oder Diethylaminogruppe bedeutet.